# EUROPEAN PATENT SPECIFICATION

(11) **EP 2 367 530 B1**
(45) Date of publication and mention of the grant of the patent: **01.05.2013**
(21) Application number: 09795930.8
(22) Date of filing: 18.12.2009
(51) Int. Cl.: A61K 9/08, A61P 7/08

(54) **SOLUTIONS FOR VOLUME THERAPY**
LÖSUNGEN FÜR DIE VOLUMENTHERAPIE
SOLUTIONS POUR THÉRAPIE DE VOLUME

(30) Priority: 19.12.2008 EP 08022089
(43) Date of publication of application: 28.09.2011
(73) Proprietor: Fresenius Kabi Deutschland GmbH, 61352 Bad Homburg (DE)
(72) Inventor: BREITER, Norbert, 82166 Gräfelfing (DE)
(86) International application number: PCT/EP2009/009123
(87) International publication number: WO 2010/078935

(56) References cited:
- WO-A1-2005/014025
- WO-A2-2004/034887
- US-A1- 2004 082 022

## Description

### Technical Field

The invention relates to artificial plasma-like solutions and methods for their use. The subject solutions find use in a variety of applications, particularly in those applications where at least a portion of a host's blood volume is replaced with a blood substitute.

### Background Art

Physiologically acceptable solutions find use in a variety of different applications in the medical, biomedical research and related fields. For example, physiologically acceptable solutions find use as plasma substitutes in surgical applications which require the replacement of significant amounts of blood plasma volume. Such applications include the treatment of blood loss during surgery or trauma, or when a tissue, organ, group of organs or an entire subject needs to be maintained at a hypothermic or frozen state. Such applications also include applications in which a patient's blood is flowed through an external device, such as a cardiopulmonary bypass machine, where the extra circulatory volume space resulting from attachment of the patient's circulatory system to the device must be filled with a compatible blood substitute, i.e., blood volume expander.

Physiologically acceptable solutions suitable for use as plasma expanders/substitutes must be able to mix freely with blood without unacceptably compromising its components, such as creating precipitates which significantly block flow in small vessels, destroying an unacceptable portion of its formed elements (cells, platelets), introducing agents or creating water, ionic or molecular imbalances destructive to body cells and tissues, or causing harmful physiologic activities such as inappropriate acceleration or inhibition of heartbeat, nerve conduction or muscle contraction, and the like. The first plasma substitute solutions employed were derived from mammalian blood. Although such solutions have been used with success, because such solutions are derived from natural blood, they can contain various pathogenic substances, such as viral pathogens such as HIV, Hepatitis B, and other pathogens, e.g., prions such as those associated with Creutzfeldt-Jakob disease, and the like. As such, use of blood substitutes and plasma substitute solutions derived from natural blood are not free of complications.

As such, a variety of synthetic blood and plasma substitute solutions have been developed which are prepared from non-blood derived components. Although synthetic plasma-like solutions have found increasing use in a variety of applications, no single solution has proved suitable for use in all potential applications without side effects. Accordingly, there is continued interest in the development of new physiologically acceptable aqueous solutions that are suitable for use as plasma substitutes, mimicking the physiological properties of blood. Of particular interest is the development of solutions that are suitable for use in hypothermic surgical applications, such as cardiac surgery and the like.
WO 2004/034887 describes a method for reducing the intracellular lipid storage material of a cell, tissue, or organ for transplantation and features solutions, methods and kits that induce the metabolic elimination of lipid storage in a cell, tissue, or organ.
WO 2005/014025 describes a stable pharmaceutical solution preparation of erythropoietin (EPO), which includes a stabilizing agent not containing a blood-derived protein, thereby maintaining EPO activity for a prolonged period of time without the risk of viral contamination.
Finally, US 2004/082022 describes an artificial plasma-like substance having at least one water soluble polysaccharide oncotic agent selected from the group consisting of high molecular weight hydroxyethyl starch, low molecular weight hydroxyethyl starch, dextran 40 and dextran 70, which is buffered by lactate and has a pre-administration pH of between 4 and 6.5.

Therefore the object of the present invention was to provide new physiologically acceptable aqueous solutions that are suitable for use as blood volume expanders. These solutions should mimic the physiological properties of blood.

### Disclosure of Invention

Said object has been achieved by the present invention, according to claim 1.

The invention provides colloids, preferably hydroxyethyl starches, in specific solutions disclosed below, useful as blood volume expanders.

In EP1164841 non-phosphate-buffered liquid cell culture media are disclosed. It has now been surprisingly found that solutions disclosed therein serve as an improved carrier solution for colloids, especially for hydroxyethyl starches.

The terms "blood volume expander", or "blood volume expander solution", or "blood substitute" as used herein mean a physiological liquid solution which is intended for use in replacing, expanding or maintaining blood volume. These solutions therefore find use in substituting for a loss of blood volume resulting from hypovolemia.

The term "hypovolemia" as used herein means a state of decreased blood volume, or more specifically, a state of decreased blood plasma volume. Common causes of hypovolemia include dehydration, burns, bleeding or the intake of certain drugs such as diuretics and vasodilators.

In one embodiment, the solutions will include a plurality of electrolytes, including: sodium ions, chloride ions, potassium ions and optionally calcium ions. The sodium ion concentration of the solutions will range from about 70 to 160 mmol/L, usually from about 100 to 150 mmol/L, and and will be preferably about 110 mmol/L. In another prefered embodiment, the sodium ion concentration is 135 mmol/L.

The concentration of chloride ion in the solution will range from about 70 to 180 mmol/L, usually from about 80 to 160 mmol/L, more usually from about 100 to 135 mmol/L and in some embodiments from about 110 to 125 mmol/L.

The concentration of potassium ion will range from about 0.1 to 6.2 mmol/L, usually from 2.5 to 6.2 mmol/L, more usually from about 4.0 to 5.5 mmol/L, where in many embodiments of the invention, the potassium ion concentration will range from about 1 to 5.5 mmol/L, usually from about 4.5 to 5.5 mmol/L, where in certain embodiments, the potassium ion concentration may be higher than 5.5 mmol/L and range as high as about 6.2 mmol/L or higher, but will usually not exceed about 6.2 mmol/L. Most preferably, the potassium ion concentration is 5 mmol/L.

The solutions may also include calcium ions. The calcium ion concentration will be ranging from about 0 to 6.0 mmol/L, and in many embodiments will range from about 0.1 to 4.0 mmol/L, usually from about 0.1 to 2.5 mmol/L, preferably from 1.1 to 1.4 mmol/L, more preferably from 1.2 to 1.3 mmol/L, even more preferably about 1.25 mmol/L.

The solutions may further include magnesium ions. The magnesium ion will range from about 0 to 10 mmol/L, usually from about 0.2 to 3.0 mmol/L and more usually from about 0.3 to 0.5 mmol/L. Preferably, the solution comprises about 0.45 mmol/L magnesium ions.

In one preferred embodiment, the solution contains a concentration ratio of calcium ions and magnesium ions of 5:1 - 1:1, preferably in a concentration ratio of from 4:1 to 2:1, more preferably about 3:1.

Chloride ions are preferably provided as sodium, potassium, calcium and magnesium salts. Preferably, when present, choline is also provided as a chloride salt.

The solutions also include a colloid. The colloid includes molecules whose size is sufficient to prevent its loss from the circulation by readily traversing the fenestrations of the capillary bed into the interstitial spaces of the tissues of the body. As a group, colloids are exemplified by blood plasma expanders. Compounds finding use as colloids in the subject invention may be natural or synthetic, and will usually be polymeric compositions having an average molecular weight of at least about 40,000 Da, usually at least about 100,000 Da, where colloids having a molecular weight of 300,000 Da or higher may find use.

Hydroxyethyl starches are of particular interest for the subject invention by forming preferred colloids. Hydroxyethyl starch (HES) types are physicochemically characterized by their mean molecular weight, molar substitution and C2/C6 ratio. Molar substitution is defined as the number of hydroxyethyl residues per glucose subunit, while the C2/C6 ratio describes the hydoxyethylation pattern of the carbon atoms in the glucose ring. HES compounds can be derived from different vegetable sources such as waxy maize, pea and potato.

The weight average mean molecular weight of hydroxyethyl starches finding use in the subject invention may range from 10,000 to 1,000,000 Dalton (Da) or higher, where the molecular weight will typically range from about 40,000 to 1,000,000 Da, usually from about 100,000 to 900,000 Da, and more usually from about 200,000 to 800,000 Da. Preferred are compositions in which the average molecular weight of the hydroxyethyl starch ranges from about 50,000 to 1,000,000 Da, usually from about 100,000 to 900,000 Da and more usually from about 100,000 to 800,000 Da. The degree of substitution will range from about 0.1 to 10, where in certain embodiments, the degree of substitution will range from 7 to 10, in other embodiments will range from 3 to 5, and in other embodiments will range from 6 to 7.

Therefore, one class of preferred solutions will include a hydroxyethyl starch with between about 6 and 7 hydroxyethyl groups for every 10 glucose units. Another class of preferred solutions will include between about 4 and 5 hydroxyethyl groups for every 10 glucose units. Yet another class of preferred solutions will include between about 7 and 8 hydroxyethyl groups for every 10 glucose units.

A particularly preferred colloid is Hetastarch (Fresenius Kabi), an artificial colloid derived from a waxy maize starch composed almost entirely of amylopectin with hydroxyethyl ether groups introduced into the alpha (1,4) linked glucose units and having a molar substitution of about 0.7 hydroxyethyl groups/glucose unit. The average molecular weight of hydroxyethyl starch is 480,000 Da with a range of 400,000 to 550,000 Da and with 80% of the polymers falling in the range of 30,000 to 2,400,000 Da.

Another particularly preferred oncotic agent is Pentastarch, which has a molar substitution of about 0.5 hydroxyethyl groups/glucose unit and an average molecular weight range of from about 150,000 to 350,000 Da, with 80% between 10,000 and 2,000,000 Da.

Another particularly preferred oncotic agent is "Hexastarch," which has a molar substitution of about 0.62 hydroxyethylgroups/glucose unit and an average molecular weight of about 220,000 Da.

Another particularly preferred oncotic agent is "Tetrastarch", which has a molar substitution of about 0.4 hydroxyethylgroups/glucose unit and an weight average mean molecular weight of about 130,000 Da.

In certain embodiments, the hydroxyethyl starch will be a select fraction of the initial hydroxyethyl starch source, particularly a select size fraction, where generally the fraction will be at least one of the fraction having an average molecular weight of less than about 1,000,000 Da or the fraction having an average molecular weight of greater than about 50,000 Da. Conventional fractionation means may be used to prepare such fractions. The concentration of oncotic agent in the solution is sufficient to achieve (when taken together with chloride salts of sodium, calcium and magnesium, organic ion from the organic salt of sodium and hexose sugar discussed above) colloid osmotic pressure approximating that of normal human serum, about 28 mm Hg.

For preparation, analytics and properties of hydroxyethyl starches see Sommermeyer, Cech, Schmidt et al, Chromatographia 1988, 25, 167-168; Jungheinrich and Neff, Clin Pharmacokin 2005, 44(7), 681-699; John Milton Mishler IV, Pharmacology of hydroxyethyl starches (1982) Oxford Medical Publications, p. 1-30.

Generally, the amount of colloid in the solution will range from about 0.5 to 30 %, usually from about 0.5 to 25 % and more usually from about 0.5 to 8 %. Where the colloid is a hydroxyethyl starch, the amount present in the solution will range from about 0.5 to 30 %, usually from about 0.5 to 15 % and more usually from about 0.5 to 8%.

Surprisingly it has been found that the addition of specific amino acids have a strong impact on the physiological compatibility of the solution. Especially glutamine has been found to have beneficial effects on the solution when used as a plasma expander. Therefore the solution contains glutamine in an amount of at least 100 µmol/L.

In a further embodiment the solution contains from 100 - 2000 µmol/L glutamine, preferably about 400 µmol/L.

The solution may also contain a buffer, where the term buffer is used to refer to one or more reagents that work to keep the pH of the solution in a certain range in an in vivo environment. The buffer, if present, is not an inorganic phosphate buffer.

Preferably the non inorganic phosphate buffer has a pKa value in aqueous solution of 7.1 to 7.5 at 20°C.

In one embodiment, the non-phosphate buffer is present at a concentration of from 1 to 12 mmol/L, preferably 3 to 7 mmol/L, more preferably 4 to 6 mmol/L and most preferably about 5 mmol/L.

Preferably the buffer is selected from the group consisting of 2-(N-morpholino)ethanesulfonic acid (MES), N-(2-acetamido)iminodiacetic acid (ADA), N-2-(acetamido)-2-aminoethanesulfonic acid (ACES), N,N-bis(2-hydroxyethyl)glycine (BICINE), piperazine-N,N'-bis(2-ethanesulfonic acid) (PIPES), N-tris(hydroxymethyl)methylglycine (TRICINE), N-2-Hydroxyethylpiperazine-N'-2-hydroxypropanesulfonic acid (HEPES), 3-(N-Morpholino) propanesulfonic acid (MOPS), 2-([2-Hydroxy-1,1-bis(hydroxymethyl)ethyl]amino)ethanesulfonic acid (TES), 3-[N-tris(Hydroxy-methyl)ethylamino]-2-hydroxyethyl]-1-piperazinepropanesulfonic acid (EPPS), N,N-bis(2-hydroxyethyl)-2-aminoethanesulfonic acid (BES), Tris[hydroxymethyl]-aminomethane (THAM), and Tris[hydroxymethyl]methyl aminomethane (TRIS), or combinations therof. Most preferably the buffer is selected from the group consisting of 3-(N-Morpholino) propanesulfonic acid (MOPS), 2-([2-Hydroxy-1,1-bis(hydroxymethyl)ethyl]amino)ethanesulfonic acid (TES) and N,N-bis(2-hydroxyethyl)-2-aminoethanesulfonic acid (BES), or combinations thereof.

In one embodiment, the solution takes advantage of a natural buffer system. Preferably this buffering system takes the form of a natural physiological buffer system, namely NaHCO₃/pCO₂, has been adopted for the solution in accordance with the invention, in combination with the zwitterionic Good's buffer, BES (Good et al. Biochemistry 5 : 467-477), which acts by virtue of its ideal pKa over a temperature range of 10-37° C, to provide a stable pH. BES has been shown to be non-toxic to even cultured mammalian cells in longterm studies and exhibits negligible binding of Ca²⁺ or Mg²⁺, so removing the potential hazard of precipitation of divalent ions which occurs when using conventional bicarbonate/phosphate or double phosphate buffer solutions. As alternatives to the use of N, N-bis-(2-hydroxyethyl)-2-amino ethanesulfonic acid (BES), it is possible to use morpholinopropane sulfonic acid (MOPS) or N-trishydroxymethyl) methyl-2-amino ethanesulfonic acid (TES).

Preferably, the solution contains 18 to 40 mmol/L, more preferably 21 to 35 mmol/L, most preferably about 25 mmol/L bicarbonate ions.

Aspartate and glutamate may also been included in solutions according to the invention to enhance oxidative metabolism by replenishing TCA cycle intermediates, thereby maintaining high energy phosphate levels even during ischemic insult. Similarly, glutamate is involved in maintaining intracellular oxidation-reduction potentials. Essentially, it is suggested that, by optimising the aspartate-malate and glycerol phosphate shuttles, cells will maintain an optimal NAD/NADH balance and thereby sustain adenine nucleotide levels.

The amino acid component may comprise glutamate, in particular L-glutamate, from 5 to 400 µmol/L, in particular about 300 µmol/L.

The amino acid component may also comprise aspartate, in particular L-aspartate, from 5 to 200 µmol/L, in particular 20 µmol/L.

Thiamine cocarboxylase plays an essential role in the oxidation of a-keto acids, and is included in the compositions to prevent the accumulation of pyruvate and pyruvate aldehyde and thereby cell toxicity.

In the tricarboxylic acid cycle, TPP is a co-factor in the metabolism of a-ketoglutaric acid to form succinyl-coenzyme A, by oxidative decarboxylation, or to form glutamate, by reductive amination.

In essence, TPP is involved in numerous interrelated, biochemical pathways, especially those of the Pentose Phosphate and Glycolytic pathways.

The thiamine may, for instance, be employed as thiamine pyrophosphate or as thiamine diamide.

Preferably the solution comprises 1 to 120 nmol/L of thiamine, in particular as thiamine pyrophosphate chloride, more preferably about 40 nmol/L of thiamine, in particular thiamine pyrophosphate chloride.

The vitaminoid carnitine has been reported to have multiple effects in improving cardiac function other than by simply optimising oxidative metabolism, such as, by promoting the utilization of alternative substrates and may additionally improve coronary blood flow. L-carnitine is preferred to the D-or DL-isomers, because it causes no inhibition of acetyl coenzyme A/free fatty acid metabolism. Preferably the vitaminoid component comprises from 40 to 70 µmol/L, in particular 50 µmol/L of carnitine, in particular [-]-3-hydroxy-y-trimethylamino-butyrate hydrochloride (L-carnitine). In this invention the inclusion of the L-isomer of carnitine in the formulation of the solution was intended to optimise the transport of long chain fatty acids from the cytosol into the mitochondrial matrix to the site of (3-oxidation and thereby to buffer the intramitochondrial acetyl CoA/CoA ratio by stimulating the synthesis of acetyl carnitine from carnitine acetyltransferase. This reduction in the ratio of acetyl CoA/CoA will result in an efflux of acetyl carnitine from the mitochondria with an associated stimulation of pyruvate dehydrogenase and reversal of fatty acid inhibition of glucose oxidation. Ultimately, the optimisation of free fatty acid utilisation as an energy source is essential for all types of cells but this must be done with preservation of carbohydrate (glucose) utilisation by optimised functioning of the enzymes involved in glycolysis, eg. hexokinase, glucokinase, phosphofructokinase.

The use of human recombinant insulin not only precludes the risk of antigenic or viral contamination in recipient cells/tissues/organs, as may be the case with insulin derived from other mammalian or animal species, but leads to a better fit being achieved of insulin molecules to human insulin receptor structure, i.e. receptor specificity will be optimised to retain the many associated functions of insulin in cellular processes.

The solution optionally comprises from 5 to 200 ml.U./L, preferably from 5 to 100 ml. U./L, most preferably about 28 ml.U./L of recombinant human insulin, for instance expressed in E. coli.

Glucose, glycerol and choline are optionally added to the solution. Apart from their ability to be metabolised, glycerol and glucose also have free radical scavenging and membrane stabilizing properties, which have been shown to be important. Preferably, the substrate component comprises from 2 to 15 mmol/L, preferably from 2 to 11 mmol/L and more preferably about 10 mmol/L of glucose, in particular D-glucose; from 50 to 150 µmol/L, preferably about 110 µmol/L of glycerol; and from 7 to 15 µmol/L, preferably about 10 µmol/L of choline, in particular L-choline, preferably as the chloride salt.

The solution may also contain from 15 to 215 µmol/L, preferably about 60 µmol/L pyroglutamate and/or from 20 to 200 µmol/L, preferably about 100 µmol/L of arginine, preferably L-arginine.

The solution may also contain other components which are physiologically acceptable.

The solutions of the invention find use in methods for treating hypovolemia or the loss of interstitial and extracellular fluid brought about in subjects suffering from severe burns. In addition, the solutions of the invention are applicable in methods of preventing and/or ameliorating reperfusion injury.

Therefore, the invention provides medicaments as defined herein for use in treating hypovolemia and/or burns.

The solution of the present invention may be administered systemically, in particular intravenously or intraarterially to a subject in need of such a treatment.

Preferred embodiments of the invention are the solutions disclosed in the following table:

**Table 1**

| | |
|---|---|
| NaCl | 100-150 mmol/L |
| KCl | 2.5-6.2 mmol/L |
| CaCl₂ | 0.1-2.5 mmol/L |
| MgCl₂ | 0.4-25 mmol/L |
| NaHCO₃ | 21 to 35 mmol/L |
| BES | 3-7 mmol/L |
| D-Glucose | 2-11 mmol/L |
| Glycerol | 0.05-0.15 mmol/L |
| L-Glutamate | 0.005-0.4 mmol/L |
| L-Glutamine | 0.1-2 mmol/L |
| L-Aspartate | 0.005-0.2 mmol/L |
| L-Camitine | 0.04-0.07 mmol/L |
| Choline Chloride | 0.007-0.015 mmol/L |
| Thiaminpyrophosphate | 1-120 nmol/L |
| Human recombinant insulin | 5-200 mlU/L |
| Hydroxyethyl starch (mean molecular weight 100,000 - 500,000 Da) | 0.5 - 8% |

More preferred embodiments of the invention are the solutions disclosed in the following table:

**Table 2**

| | |
|---|---|
| NaCl | about 110 mmol/L |
| KCI | about 5 mmol/L |
| CaCl₂ | about 1.25 mmol/L |
| MgCl₂ | about 0.45 mmol/L |
| NaHCO₃ | about 25 mmol/L |
| BES | about 5 mmol/L |
| D-Glucose | about 10 mmol/L |
| Glycerol | about 0.11 mmol/L |
| L-Glutamate | about 0.3 mmol/L |
| L-Glutamine | about 0.4 mmol/L |
| L-Aspartate | about 0.02 mmol/L |
| L-Carnitine | about 0.05 mmol/L |
| Choline Chloride | about 0.01 mmol/L |
| Thiaminpyrophosphate | About 40 nmol/L |
| Human recombinant insulin | About 28 mlU/L |
| Hydroxyethyl starch (mean molecular weight 100,000 - 500,000 Da) | 0.5 - 6% |

These solutions mimic the physiological properties of blood in an exceptional way and are therefore particularly appropriate for the treatment of blood loss.

These and other aspects of the invention will be apparent from and elucidated with reference to the embodiments described hereinafter.

### Brief Description of Drawings

Fig. 1 shows the mean heart rate of rats in response to administration of the inventive physiologically acceptable aqueous solution after induction of a shock by withdrawal of blood and in comparison to three other solutions.

Fig. 2 shows the mean arterial blood pressure of rats in response to administration of the inventive physiologically acceptable aqueous solution after induction of a shock by withdrawal of blood and in comparison to three other solutions.

### Examples

The following examples demonstrate the superiority of the solutions according to the present invention in comparison to standard solutions. Particularly it has surprisingly been found that rats suffering from severe blood loss infused with the solution disclosed above show a higher survival rate and/or longer survival time than rats treated with standard therapy, e. g. infusion of 0.9% NaCl (aq) solution or Ringer's lactate solution. In addition, administration of the solutions according to the invention positively affected blood pressure and heart rate of these rats.

### Preparation of a typical solution containing a colloid according to the invention

EP1164841 discloses the preparation of a typical basic solution without colloid according to the invention:

Preparation of the basic solution: Thiamine pyrophosphate (cocarboxylase), Sigma C4655 was prepared as a 0. 4 mg/mL stock solution in MilliQ (endotoxin-free) purified water, and stored frozen in dark glass vials. Choline chloride (Sigma C7527) was prepared as a 17. 5 mg/mL stock solution in MilliQ endotoxin-free purified water and stored frozen in glass vials. Human recombinant insulin (Sigma 10259) was prepared as a 0. 5 I. U./mL stock solution in endotoxin-free MilliQ purified water acidified to pH 2. 4 with 0. 1N hydrochloric acid and stored frozen in glass vials.

In the following preparations, endotoxin-free MilliQ purified water was used throughout, both in the initial stirring, and in the final dilution.

For the preparation, a stainless steel container was filled with 8 litres of MilliQ and, the following ingredients were weighed out and added while constantly stirring, in the following order: 642.96 grams of sodium chloride, 37.28 grams of potassium chloride, 18.38 grams of calcium chloride dihydrate, 9.14 grams of magnesium chloride hexahydrate and 106.61 grams of BES free acid (Sigma B6266), 1.84 milligrams of thiamine pyrophosphate (Sigma C9655) (using 4. 6 mL of the stock solution), 0.9899 grams of L-carnitine (Sigma C0238), 0.1397 grams of choline chloride (Sigma 7527) in the form of 8 ml of the stock solution, 1.013 grams of glycerol (Sigma G2025), 2.8 I. U. of human recombinant insulin (5 ml of the stock solution), 0.310 grams of L-aspartate sodium salt (Sigma A6683), 180.2 grams of anhydrous D-glucose (Sigma G7021), 5.07 grams of L-glutamate sodium salt (Sigma G5889) and 5.84 grams of L-glutamine (Sigma G5763). The whole was stirred until completely dissolved and then the final volume of 10 litres was produced by adding further MilliQ purified water.

The solution was filtered through a sterile filter (0.2 um Sartobran PH) into 100 mL sterile sealed glass bottles.

This solution is a 10x concentrate of the solution intended for use. When needed, it can be diluted with the appropriate quantity of MilliQ.

100 mL of the concentrate may be diluted with 900 mL of double deionised or endotoxin-free MilliQ purified water to 1 litre with the addition of 2.1 g of endotoxin-free sodium bicarbonate (Sigma S4019) and stored at 8-10 C prior to use. Sodium bicarbonate is not added to the concentrates before they are stored, since extended storage of the concentrate containing bicarbonate ions may cause precipitations of calcium carbonate.

The colloid may be dissolved in the solution before sterile filtration of the 10x concentrate in said process. Alternatively the colloid may be added via a sterile aqueous solution in the dilution step which follows the sterile filtration of the basic solution.

### Rat model

The aim of this study was the comparison of the following test solutions in a standardized rat model of hemorrhagic shock: 1) The basic "Aqix", solution, commercially available from AQIX Ltd, UK, 2) A 6% hydroxyethyl starch solution HES 130/0.4 (Voluven, obtainable from Fresenius Kabi), 3) A 6% hydroxyethyl starch solution of HES 130/0.4 diluted in Aqix, and 4) A Ringer's lactate preparation.

Aqix and the 6% HES 130/0.4 solution were obtained as cristalloid solution from AQIX Ltd and from Fresenius Kabi, respectively. For the 6% HES 130/0.4 HES in Aqix-solution 15 g HES powder was diluted in 250ml Aqix solution. The standard Ringer's Lactate-Solution was obtained from B. Braun.

A number of 52 male rats (RjHan:SD) of 330 - 370 g bodyweight, bred by Elevage Janvier Le Genest St Isle (France), was used as test species and grouped into four groups corresponding to the four test solutions as defined above:

**Table 3**

| **Test Solutions** | **Administration** |
|---|---|
| 1) Aqix | 13 rats |
| 2) HES 130/0.4 | 13 rats |
| 3) HES 130/0.4 in Aqix | 13 rats |
| 4) Ringer's Lactate | 13 rats |
| **Total number** | **52 rats** |

A central venous catheter and an arterial catheter were inserted surgically after the rats had been anesthetised with Ketamine/Xylazine/Midazolam (100mg/10mg/1mg per kg bodyweight) administered intraperitoneally for induction of anaesthesia and administration of Pentobarbital (16 mg/ml) for the maintenance of the anaesthesia.

After stabilisation of the rats, 50 % of the rats' blood volume was withdrawn from the arterial catheter in order to induce a shock. Afterwards, the test solutions were administered to the rats by intravenous infusion via the central venous catheter.

The blood samples of 50% were withdrawn from the arterial catheter at 0.5 ml/min and following a two-step procedure: Initially, 36% of the blood volume was withdrawn and after an intermission of 10 minutes a further 14% of the blood volume was withdrawn.

Following the withdrawal of the blood samples, the rats were allowed 5 minutes for recovery after which the testing solutions were infused via the jugular catheter und using automatic infusion pumps (Infusomat fm, Braun-Melsungen, Melsungen):

In case of solutions 1) and 4) (Ringer's lactate and Aqix), a 1.38-fold amount of the blood volume withdrawn was infused at 1.0 ml/min.

In case of solutions 2) and 3) (HES 130/0.4 and HES 130/0.4 in Aqix) an equivalent amount of the blood volume withdrawn was infused at 0.5 ml/min. After the experiment wounds were closed and animals could recover from anaesthesia.

### Haemodynamic Parameters

Arterial blood pressure and heart rate were measured as haemodynamic parameters via the arterial catheter and using a blood pressure monitor (plug-sys, available from Hugo Sachs, Freiburg, Germany). Measurements were taken before the induction of the shock by blood removal (time point [TP] 1), after blood removal (time point 2), before infusion (time point 3), after infusion (time point 4) and every 30 minutes after infusion over 2 hours.

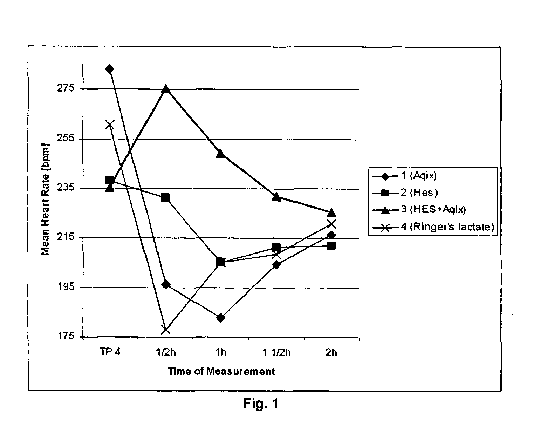

The monitoring of the mean heart rate (MHR) of the rats resulted in the values depicted in the following Table 4 and shown in Fig. 1.

**Table 4**

| **Group** | TP 1 | TP 2 | TP 3 | TP 4 | 0.5h | 1h | 1.5h | 2h |
|---|---|---|---|---|---|---|---|---|
| **1** | 359.38 | 162.65 | 178.37 | 282.99 | 196.27 | 182.95 | 204.41 | 216.63 |
| **2** | 319.23 | 175.19 | 176.78 | 238.03 | 231.20 | 205.32 | 211.23 | 212.09 |
| **3** | 330.77 | 174.78 | 182.50 | 235.18 | 275.57 | 249.28 | 231.83 | 225.46 |
| **4** | 305.14 | 170.03 | 153.00 | 260.71 | 177.92 | 205.31 | 208.42 | 221.03 |

Group numberings correspond to the grouping of test animals defined in Tab. 3: 1= Aqix solution; 2 = HES 130/0.4 solution; 3 = HES 130/0.4 in Aquix solution; 4 = Ringer's lactate. TP = time point as defined above.

The highest MHR values were observed before withdrawal of blood (TP1). In state of shock (TP2, TP3) induced by the blood withdrawal a pronounced decrease of the heart rate in all groups could be detected.

As can be taken from the measured values, test solution 3, i.e. the solution according to the invention containing a colloid, clearly demonstrates superior effects with regard to the MHR of the rats after the induction of a shock. It is shown that after 30 minutes of the induction of the shock the MHR of the rats that received test solution 3 is the highest compared to the three remaining groups. Rats that had received solution 3, clearly showed an improved haemodynamic stability.

Furthermore, if the MHR values for TP 4 are compared to the 0.5 h values, it becomes evident that only the inventive solution 3 results in an actual elevation of the MHR directly after the induction of the shock. This elevated MHR brought about by the inventive solution is maintained over the remaining course of the experiment as the MHR of the rats in group 3 continues to be the highest throughout the experiment.

The overall decrease in the MHR, i.e. at 1h, 1.5h and 2h after the infusion compared to 0.5 h after infusion, is most likely due to the fact that the infused substances are continuously excreted from the rats' system. However, the solution according to the invention containing a colloid clearly shows a prolonged retention time and/or an improved effect.

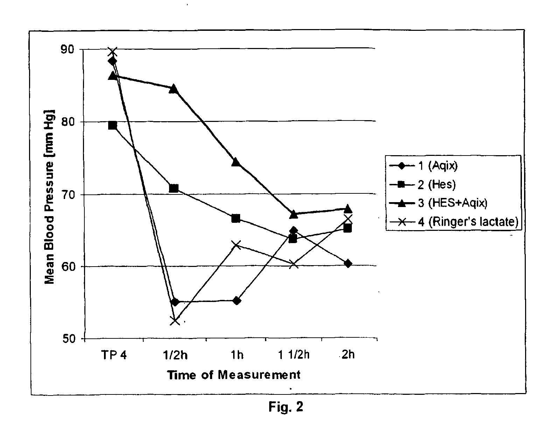

The monitoring of the mean arterial pressure (MAP) of the rats resulted in the values depicted in the following Table 5 and shown in Fig. 2.

**Table 5**

| **Group** | TP 1 | TP 2 | TP 3 | TP 4 | 0.5h | 1h | 1.5h | 2h |
|---|---|---|---|---|---|---|---|---|
| **1** | 110.17 | 36.47 | 23.15 | 88.36 | 55.00 | 55.19 | 64.86 | 60.22 |
| **2** | 104.10 | 40.90 | 23.06 | 79.40 | 70.74 | 66.52 | 63.75 | 65.15 |
| **3** | 112.36 | 30.66 | 21.34 | 86.36 | 84.58 | 74.39 | 67.16 | 67.80 |
| **4** | 104.73 | 38.52 | 29.79 | 89.67 | 52.41 | 62.92 | 60.16 | 66.38 |

Group numberings correspond to the grouping of test animals defined in Tab. 3: 1= Aqix solution; 2 = HES 130/0.4 solution; 3 = HES 130/0.4 in Aquix solution; 4 = Ringer's lactate. TP = time point as defined above.

The observed values for MAP decreased to values of approximately 20 to 30 mm HG after the withdrawal of 50 % of total blood volume (TP 3), clearly indicating a severe state of shock.

As with the MHR, also the measured values for the MAP clearly indicate that test solution 3, i.e. the solution according to the invention containing a colloid, counteracts the effects of the induced shock better than any of the other tested solutions and showed an improved haemodynamic stability. After infusion of the solution MAP recovered almost to the base level.

Again, it became evident that after 30 minutes of the induction of the shock the MAP of the rats that received test solution 3 was the highest compared to the three remaining groups. Particularly, if the MAP values for TP 4 are compared to the 0.5 h values, it becomes clear that only the inventive solution 3 averts the radical drop of the MAP right after the induction of the shock and maintains an elevated MAP over the remaining course of the experiment, as the MAP of the rats in group 3 continues to be the highest throughout the experiment.

As with the MHR, the overall decrease in the MAP, i.e. at 1h, 1.5h and 2h after the infusion compared to 0.5 h after infusion, is most likely due to the fact that the infused substances are continuously excreted from the rats' system. However, even after 1 hour of infusion the difference in the MAP - unexpectedly even between group 3 and group 2 - was statistically significant. Thus, the solution according to the invention containing a colloid clearly shows a prolonged retention time and/or an improved effect.

### Survival Rates

Long-term recovery of rats from groups 2) - 4), i.e. the standard 6% hydroxyethyl starch solution HES 130/0.4, the 6% hydroxyethyl starch solution of HES 130/0.4 diluted in Aqix and the Ringer's lactate preparation, respectively, was observed for 10 days. Animals were inspected two times a day. Animals lying in lateral position were supposed to be killed.

The highest survival rate of more than about 80 % was found in group 3), i.e. in the group of rats that had received the solution according to the invention containing a colloid. Contrasting this, the lowest survival rate of less than about 60 % was found in group 4), i.e. in the group of rats that had received the Ringer's lactate solution. Thus, the solution according to the invention containing a colloid clearly shows superior effects with regard to the survival rates of the treated animals.

In the claims, the word "comprising" does not exclude other elements or steps, and the indefinite article "a" or "an" does not exclude a plurality.

## Claims

1. A physiologically acceptable aqueous solution for use in the treatment of hypovolemia and/or burns comprising: electrolytes, selected from the group consisting of sodium, potassium, and optionally calcium and/or magnesium ions; glutamine in a concentration of at least 100 µmol/L; and at least one colloid in a concentration range from 0.5 to 30%.

2. A physiologically acceptable aqueous solution according to claim 1 for use in the therapy according to claim 1, where the sodium ion concentration of the solutions ranges from 70 to 160, preferably is 110 mmol/L, where the concentration of potassium ions ranges from 2.5 to 6.2, preferably is 5.0 mmol/L, where the calcium ion concentration ranges from 0 to 6.0 mmol/L, preferably is 1.25 mmol/L and where the magnesium ion will range from 0 to 10 mmol/L, preferably is 0.45 mmol/L.

3. A physiologically acceptable aqueous solution according to any of claims 1 or 2 for use in the therapy according to claim 1, where the solution contains a buffer which is not a phosphate inorganic buffer.

4. A physiologically acceptable aqueous solution according to claim 3 for use in the therapy according to claim 1, where the buffer is selected from the group consisting of 3-(N-Morpholino) propanesulfonic acid (MOPS), 2-([2-Hydroxy-1,1-bis(hydroxymethyl)ethyl]amino)ethanesulfonic acid (TES) and N,N-bis(2-hydroxyethyl)-2-aminoethanesulfonic acid (BES).

5. A physiologically acceptable aqueous solution according to any of claims 1 to 4 for use in the therapy according to claim 1, where the colloid is a hydroxyethyl starch.

6. A physiologically acceptable aqueous solution according to claim 5 for use in the therapy according to claim 1, where the amount of hydroxyethyl starch will range from 0.5 to 30, preferably from 0.5 to 15 and more preferably from 0.5 to 8%.

7. A physiologically acceptable aqueous solution according to any of claims 1 to 6 for use in the therapy according to claim 1, comprising 18 to 40 mmol/L, more preferably 21 to 35 , most preferably about 25 mmoles bicarbonate ions.

8. A physiologically acceptable aqueous solution according to any of claims 1 to 7 for use in the therapy according to claim 1, comprising glutamate, in particular L-glutamate, from 5 to 400 µmol/L, in particular 300 µmol/L and/or aspartate, in particular L-aspartate, from 5 to 200 µmol/L, in particular 20 µmol/L.

9. A physiologically acceptable aqueous solution according to any of claims 1 to 8 for use in the therapy according to claim 1, comprising 10 to 120 nmol/L of thiamine, in particular as thiamine pyrophosphate chloride, more preferably 40 nmol/L of thiamine, in particular thiamine pyrophosphate chloride.

10. A physiologically acceptable aqueous solution according to any of claims 1 to 9 for use in the therapy according to claim 1, comprising 40 to 70 µmol/L, in particular 50 µmol/L of carnitine, in particular L-carnitine.

11. A physiologically acceptable aqueous solution according to any of claims 1 to 10 for use in the therapy according to claim 1, comprising from 5 to 200, preferably from 5 to 100, most preferably 28.0 m. I. U./L of recombinant human insulin.

12. A physiologically acceptable aqueous solution according to any of claims 1 to 11 for use in the therapy according to claim 1, comprising glucose and/or glycerol and/or choline, where the glucose, in particular D-glucose, is present in the range from 2 to 15 mmol/L, preferably from 2 to 11 and more preferably 10 mmol/L; where glycerol is present in the range from 50 to 150 µmol/L, preferably 110 µmol/L of; and where choline, in particular L-choline, is present in the range from from 7 to 15, preferably 10 µmol/L.

13. A physiologically acceptable aqueous solution according to any of claims 1 to 12 for use in the therapy according to claim 1, comprising 110 mmol/L of NaCl, 5 mmol/L of KCl, 1.25 mmol/L of CaCl₂, 0.45 mmol/L of MgCl₂, 25 mmol/L of NaHCO₃, 5 mmol/L of BES, 10 mmol/L of D-Glucose, 0.11 mmol/L of Glycerol, 0.3 mmol/L of L-Glutamate, 0.4 mmol/L of L-Glutamine, 0.02 mmol/L of L-Aspartate, 0.05 mmol/L ofL-Carnitine, 0.01 mmol/L of Choline Chloride, 40 nmol/L of Thiaminpyrophosphate, 28 mlU/L of human recombinant insulin and 0.5 - 6% of hydroxyethyl starch with an average molecular weight of 100,000 - 500,000 Da

14. A physiologically acceptable aqueous solution according to any of claims 1 to 13 for use in the therapy according to claim 1, wherein the physiologically acceptable aqueous solution is a blood volume expander.

## Patentansprüche

1. Physiologisch annehmbare wässrige Lösung zur Verwendung bei der Behandlung von Hypovolämie und/oder Verbrennungen, umfassend: Elektrolyte ausgewählt aus der aus Natrium-, Kalium- und gegebenenfalls Kalzium- und/oder Magnesiumionen bestehenden Gruppe, Glutamin in einer Konzentration von mindestens 100 µmol/l, und mindestens einem Kolloid in einem Konzentrationsbereich von 0,5 bis 30%.

2. Physiologisch annehmbare wässrige Lösung nach Anspruch 1 zur Verwendung in der Therapie nach Anspruch 1, wobei die Natriumionenkonzentration der Lösungen im Bereich von 70 bis 160 mmol/l liegt und vorzugweise 110 mmol/l beträgt, wobei die Konzentration an Kaliumionen im Bereich von 2,5 bis 6,2 mmol/l liegt und vorzugweise 5,0 mmol/l beträgt, wobei die Kalziumionenkonzentration im Bereich von 0 bis 6,0 mmol/l liegt und vorzugweise 1,25 mmol/l beträgt und wobei die Magnesiumionenkonzentration im Bereich von 0 bis 10 mmol/l liegt und vorzugweise 0,45 mmol/l beträgt.

3. Physiologisch annehmbare wässrige Lösung nach Anspruch 1 oder 2 zur Verwendung in der Therapie nach Anspruch 1, wobei die Lösung einen Puffer enthält, bei dem es sich nicht um einen anorganischen Phosphatpuffer handelt.

4. Physiologisch annehmbare wässrige Lösung nach Anspruch 3 zur Verwendung in der Therapie nach Anspruch 1, wobei der Puffer aus der aus 3-(N-Morpholino)propansulfonsäure (MOPS), 2-([2-Hydroxy-1,1-bis(hydroxymethyl)ethyl]amino)ethansulfonsäure (TES) und N,N-Bis(2-hydroxyethyl)-2-aminoethansulfonsäure (BES) bestehenden Gruppe ausgewählt ist.

5. Physiologisch annehmbare wässrige Lösung nach einem der Ansprüche 1 bis 4 zur Verwendung in der Therapie nach Anspruch 1, wobei es sich bei dem Kolloid um eine Hydroxyethylstärke handelt.

6. Physiologisch annehmbare wässrige Lösung nach Anspruch 5 zur Verwendung in der Therapie nach Anspruch 1, wobei die Menge an Hydroxyethylstärke im Bereich von 0,5 bis 30%, vorzugweise von 0,5 bis 15% und besonders bevorzugt von 0,5 bis 8% liegt.

7. Physiologisch annehmbare wässrige Lösung nach einem der Ansprüche 1 bis 6 zur Verwendung in der Therapie nach Anspruch 1, enthaltend 18 bis 40 mmol/l, besonders bevorzugt 21 bis 35 mmol/l, ganz besonders bevorzugt etwa 25 mmol/l an Hydrogencarbonationen.

8. Physiologisch annehmbare wässrige Lösung nach einem der Ansprüche 1 bis 7 zur Verwendung in der Therapie nach Anspruch 1, enthaltend Glutamat, insbesondere L-Glutamat, von 5 bis 400 µmol/l, insbesondere 300 µmol/l, und/oder Aspartat, insbesondere L-Aspartat, von 5 bis 200 µmol/l, insbesondere 20 µmol/l.

9. Physiologisch annehmbare wässrige Lösung nach einem der Ansprüche 1 bis 8 zur Verwendung in der Therapie nach Anspruch 1, enthaltend 10 bis 120 nmol/l Thiamin, insbesondere als Thiaminpyrophosphatchlorid, besonders bevorzugt 40 nmol/l Thiamin, insbesondere Thiaminpyrophosphatchlorid.

10. Physiologisch annehmbare wässrige Lösung nach einem der Ansprüche 1 bis 9 zur Verwendung in der Therapie nach Anspruch 1, enthaltend 40 bis 70 µmol/l, insbesondere 50 µmol/l, Carnitin, insbesondere L-Carnitin.

11. Physiologisch annehmbare wässrige Lösung nach einem der Ansprüche 1 bis 10 zur Verwendung in der Therapie nach Anspruch 1, enthaltend 5 bis 200, vorzugweise 5 bis 100, besonders bevorzugt 28,0 mIE/l rekombinantes Humaninsulin.

12. Physiologisch annehmbare wässrige Lösung nach einem der Ansprüche 1 bis 11 zur Verwendung in der Therapie nach Anspruch 1, enthaltend Glucose und/oder Glycerin und/oder Cholin, wobei die Glucose, insbesondere D-Glucose, im Bereich von 2 bis 15 mmol/l, vorzugweise 2 bis 11 mmol/l und besonders bevorzugt 10 mmol/l vorliegt; wobei das Glycerin im Bereich von 50 bis 150 µmol/l, vorzugweise 110 µmol/l vorliegt und wobei das Cholin, insbesondere L-Cholin im Bereich von 7 bis 15 µmol/l, vorzugweise 10 µmol/l vorliegt.

13. Physiologisch annehmbare wässrige Lösung nach einem der Ansprüche 1 bis 12 zur Verwendung in der Therapie nach Anspruch 1, enthaltend 110 mmol/l NaCl, 5 mmol/l KCl, 1,25 mmol/l CaCl₂, 0,45 mmol/l MgCl₂, 25 mmol/l NaHCO₃, 5 mmol/l BES, 10 mmol/l D-Glucose, 0,11 mmol/l Glycerin, 0,3 mmol/l L-Glutamat, 0,4 mmol/l L-Glutamin, 0,02 mmol/l L-Aspartat, 0,05 mmol/l L-Carnitin, 0,01 mmol/l Cholinchlorid, 40 nmol/l Thiaminpyrophosphat, 28 mIE/l rekombinantes Humaninsulin und 0,5 - 6% Hydroxyethylstärke mit einem durchschnittlichen Molekulargewicht von 100.000 - 500.000 Da.

14. Physiologisch annehmbare wässrige Lösung nach einem der Ansprüche 1 bis 13 zur Verwendung in der Therapie nach Anspruch 1, wobei es sich bei der physiologisch annehmbaren wässrigen Lösung um einen Blutvolumenexpander handelt.

## Revendications

1. Solution aqueuse physiologiquement acceptable pour une utilisation dans le traitement de l'hypovolémie et/ou de brûlures, comprenant : des électrolytes, choisis dans le groupe constitué par les ions sodium, potassium et éventuellement calcium et/ou magnésium ; de la glutamine selon une concentration d'au moins 100 µmol/l ; et au moins un colloïde dans un domaine de concentration allant de 0,5 à 30%.

2. Solution aqueuse physiologiquement acceptable selon la revendication 1 pour une utilisation dans la thérapie selon la revendication 1, dans laquelle la concentration en ions sodium des solutions va de 70 à 160, préférablement est de 110 mmol/l, dans laquelle la concentration en ions potassium va de 2,5 à 6,2, préférablement est de 5,0 mmol/l, dans laquelle la concentration en ions calcium va de 0 à 6,0 mmol/l, préférablement est de 1,25 mmol/l, et dans laquelle la concentration en ions magnésium va de 0 à 10 mmol/l, préférablement est de 0,45 mmol/l.

3. Solution aqueuse physiologiquement acceptable selon l'une quelconque des revendications 1 ou 2 pour une utilisation dans la thérapie selon la revendication 1, dans laquelle la solution contient un tampon qui n'est pas un tampon de phosphate inorganique.

4. Solution aqueuse physiologiquement acceptable selon la revendication 3 pour une utilisation dans la thérapie selon la revendication 1, dans laquelle le tampon est choisi dans le groupe constitué par l'acide 3-(N-morpholino)propanesulfonique (MOPS), l'acide 2-([2-hydroxy-1,1-bis(hydroxyméthyl)éthyl]amino)éthane-sulfonique (TES) et l'acide N,N-bis(2-hydroxyéthyl)-2-aminoéthanesulfonique (BES).

5. Solution aqueuse physiologiquement acceptable selon l'une quelconque des revendications 1 à 4 pour une utilisation dans la thérapie selon la revendication 1, dans laquelle le colloïde est l'amidon hydroxyéthylé.

6. Solution aqueuse physiologiquement acceptable selon la revendication 5 pour une utilisation dans la thérapie selon la revendication 1, dans laquelle la quantité d'amidon hydroxyéthylé va de 0,5 à 30, préférablement de 0,5 à 15 et plus préférablement de 0,5 à 8%.

7. Solution aqueuse physiologiquement acceptable selon l'une quelconque des revendications 1 à 6 pour une utilisation dans la thérapie selon la revendication 1, comprenant de 18 à 40 mmol/l, plus préférablement de 21 à 35, tout préférablement environ 25 mmol d'ions bicarbonate.

8. Solution aqueuse physiologiquement acceptable selon l'une quelconque des revendications 1 à 7 pour une utilisation dans la thérapie selon la revendication 1, comprenant du glutamate, en particulier du L-glutamate, de 5 à 400 µmol/l, en particulier 300 µmol/l, et/ou de l'aspartate, en particulier du L-aspartate, de 5 à 200 µmol/l, en particulier 20 µmol/l.

9. Solution aqueuse physiologiquement acceptable selon l'une quelconque des revendications 1 à 8 pour une utilisation dans la thérapie selon la revendication 1, comprenant de 10 à 120 nmol/l de thiamine, en particulier sous forme de chlorure de thiamine pyrophosphate, plus préférablement 40 nmol/l de thiamine, en particulier de chlorure de thiamine pyrophosphate.

10. Solution aqueuse physiologiquement acceptable selon l'une quelconque des revendications 1 à 9 pour une utilisation dans la thérapie selon la revendication 1, comprenant de 40 à 70 µmol/l, en particulier 50 µmol/l de carnitine, en particulier de L-carnitine.

11. Solution aqueuse physiologiquement acceptable selon l'une quelconque des revendications 1 à 10 pour une utilisation dans la thérapie selon la revendication 1, comprenant de 5 à 200, préférablement de 5 à 100, tout préférablement 28 mUI/l d'insuline humaine recombinée.

12. Solution aqueuse physiologiquement acceptable selon l'une quelconque des revendications 1 à 11 pour une utilisation dans la thérapie selon la revendication 1, comprenant du glucose et/ou du glycérol et/ou de la choline, où le glucose, en particulier du D-glucose, est présent dans la plage allant de 2 à 15 mmol/l, préférablement de 2 à 11 et plus préférablement de 10 mmol/l ; où le glycérol est présent dans la plage de 50 à 150 µmol/l, préférablement de 110 µmol/l ; et où la choline, en particulier de la L-choline, est présente dans la plage allant de 7 à 15, préférablement de 10 µmol/l.

13. Solution aqueuse physiologiquement acceptable selon l'une quelconque des revendications 1 à 12 pour une utilisation dans la thérapie selon la revendication 1, comprenant 110 mmol/l de NaCl, 5 mmol/l de KCl, 1,25 mmol/l de CaCl₂, 0,45 mmol/l de MgCl₂, 25 mmol/l de NaHCO₃, 5 mmol/l de BES, 10 mmol/l de D-glucose, 0,11 mmol/l de glycérol, 0,3 mmol/l de L-glutamate, 0,4 mmol/l de L-glutamine, 0,02 mmol/l de L-aspartate, 0,05 mmol/l de L-carnitine, 0,01 mmol/l de chlorure de choline, 40 nmol/l de thiamine pyrophosphate, 28 mUI/l d'insuline humaine recombinée et 0,5-6% d'amidon hydroxyéthylé ayant un poids moléculaire moyen de 100 000-500 000 Da.

14. Solution aqueuse physiologiquement acceptable selon l'une quelconque des revendications 1 à 13 pour une utilisation dans la thérapie selon la revendication 1, **caractérisée en ce que** la solution aqueuse physiologiquement acceptable est un expanseur du volume sanguin.
